(19) **Europäisches Patentamt**
**European Patent Office**
**Office européen des brevets**

(11) **EP 4 550 339 A1**

(12) **EUROPEAN PATENT APPLICATION**
published in accordance with Art. 153(4) EPC

(43) Date of publication:
**07.05.2025 Bulletin 2025/19**

(21) Application number: **23831367.0**

(22) Date of filing: **26.06.2023**

(51) International Patent Classification (IPC):
**G16H 10/00** (2018.01)

(52) Cooperative Patent Classification (CPC):
**G10K 15/02; A61M 21/02; G16H 10/00**

(86) International application number:
**PCT/JP2023/023525**

(87) International publication number:
**WO 2024/004926 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.06.2022 JP 2022103168**

(71) Applicants:
• **Pixie Dust Technologies, Inc.**
**Tokyo, 104-0028 (JP)**

• **Shionogi & Co., Ltd**
**Osaka-shi, Osaka 541-0045 (JP)**

(72) Inventors:
• **NAGATANI Yoshiki**
**Tokyo 104-0028 (JP)**
• **TAKAZAWA Kazuki**
**Tokyo 104-0028 (JP)**
• **KATAYAMA Haruki**
**Tokyo 104-0028 (JP)**

(74) Representative: **Plasseraud IP**
**104 Rue de Richelieu**
**CS92104**
**75080 Paris Cedex 02 (FR)**

(54) **SIGNAL PROCESSING DEVICE, CONGNITIVE FUNCTION IMPROVEMENT SYSTEM, SIGNAL PROCESSING METHOD, AND PROGRAM**

(57)      A signal processing apparatus of one embodiment of the present disclosure has an output means for outputting an acoustic signal generated by amplitude modulation, the acoustic signal having an amplitude change corresponding to the frequency of gamma waves, a memory means for storing information of the acoustic signal output by the output means, and a transmission means for transmitting a notification based on the information stored by the memory means to an apparatus external to the signal processing apparatus.

EP 4 550 339 A1

**(Cont. next page)**

FIG. 4

50

(1) Acquisition of acoustic signals

10

(2) Amplitude modulation

30

(3) Sound signal output

(4) Storage of listening history
(5) Creation of target information

PA1

70

PC2

(5) Notification of target information

# Description

[Technical field]

The present disclosure relates to a signal processing apparatus, a cognitive function improvement system, a signal processing method, and a program.

[Background Art]

There is a research report showing that inducing gamma waves in the brain of an organism by subjecting it to pulsating sound stimuli at a frequency of about 40 times per second is effective in improving the organism's cognitive function (see non-patent document 1).

Gamma waves refer to neural oscillations that capture periodic neural activity in the brain's cortex using electrophysiological techniques such as electroencephalography and magnetoencephalography, and whose frequencies fall within the gamma band (25 to 140 Hz).

Patent Document 1 discloses adjusting the volume by increasing or decreasing the amplitude of a sound wave or soundtrack to create a rhythmic stimulus that corresponds to a stimulation frequency that induces brainwave entrainment.

[Prior Art Documents]

[Patent Documents]

[Patent Document 1] JP2020-501853

[Non-patent Document]

[Non-Patent Document 1] Multi-sensory Gamma Stimulation Ameliorates Alzheimer's-Associated Pathology and Improves Cognition Cell 2019 Apr 4; 177(2):256-271.e22. doi: 10.1016/j.cell.2019.02.014.

[Summary of Invention]

[Technical Problem]

When a user who is hoping to improve his/her cognitive function is made to listen to a sound based on an amplitude-modulated acoustic signal, there are cases where a related party wants to understand the listening condition of the user. For example, a user's family members may become anxious if they cannot see that the user is hearing sounds. Medical personnel who perform treatment by listening to sounds cannot provide appropriate treatment instructions unless they can confirm whether the user (patient) is listening to the sounds. If the user has no opportunity to review the listening state when he or she forgets to listen to a sound, it may be difficult for the user to make listening to a sound a habit.

An object of the present disclosure is to provide a technology that enables related parties to understand the listening conditions of a user with respect to a sound corresponding to an amplitude-modulated acoustic signal.

[Solution to Problem]

A signal processing apparatus of one embodiment of the present disclosure has an output means for outputting an acoustic signal generated by amplitude modulation, the acoustic signal having an amplitude change corresponding to a frequency of gamma waves; a storage means for storing information on the acoustic signal output by the output means; and a transmitting means for transmitting a notification based on the information stored in the storage means to an apparatus external to the signal processing apparatus.

[Brief Description of Drawings]

FIG. 1 is a block diagram showing the configuration of an audio system according to an embodiment of the present invention.

FIG. 2 is a block diagram showing a configuration of a signal processing device according to the present embodiment.

FIG. 3 is a block diagram showing a configuration of a related party terminal according to the present embodiment.

FIG. 4 is an explanatory diagram of one aspect of the present embodiment.

FIG. 5 is a diagram showing a data structure of a listening history database according to the present embodiment.

FIG. 6 is a flowchart of an acoustic signal processing according to the present embodiment.

FIG. 7 is a flowchart of an information notification process according to the embodiment.

FIG. 8 is a diagram illustrating an example of a score function used in the information notification process of the present embodiment.

FIG. 9 is a diagram showing an example of presentation of target information in the information notification process of the embodiment.

FIG. 10 is a diagram showing an example of presentation of target information in the information notification process of the embodiment.

FIG. 11 is a diagram showing an example of presentation of target information in the information notification process of the embodiment.

FIG. 12 is a flowchart of an information notification process according to the first modified example.

FIG. 13 is a block diagram showing a configuration of an audio system according to a second modified example.

FIG. 14 is a flowchart of an acoustic signal processing according to a second modified example.

[Description of Embodiments]

Hereinafter, an embodiment of the present invention will be described in detail with reference to the drawings. In the drawings for explaining the embodiments, the same components are generally designated by the same reference numerals, and repeated explanations thereof will be omitted.

(1) Sound system configuration

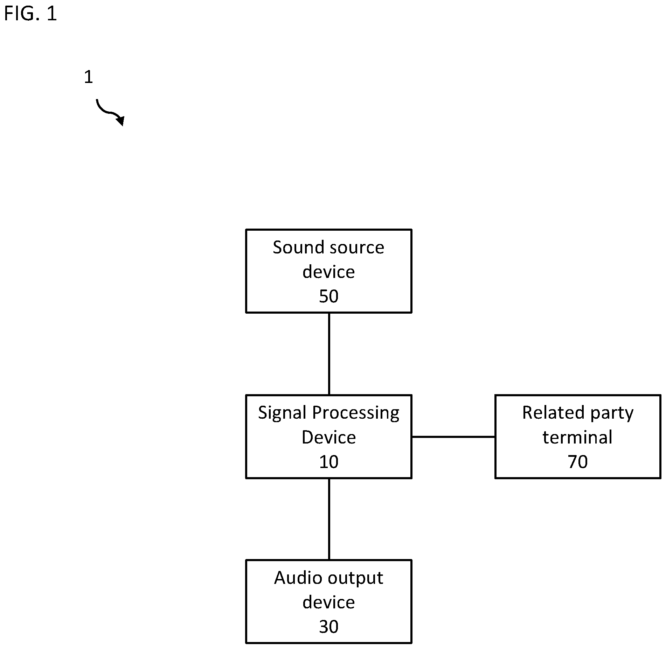

The configuration of the audio system will now be described. FIG. 1 is a block diagram showing the configuration of an audio system according to the present embodiment.

As shown in FIG. 1, the audio system 1 includes a signal processing device 10, an audio output device 30, a sound source device 50, and a related party terminal 70.

The signal processing device 10 and the sound source device 50 are connected to each other via a predetermined interface capable of transmitting an acoustic signal. The interface is, for example, a Sony Philips Digital Interface (SPDIF), a High-Definition Multimedia Interface (HDMI (registered trademark)), a pin connector (RCA pin), or an audio interface for headphones. The interface may be a wireless interface using Bluetooth (registered trademark) or the like. Similarly, the signal processing device 10 and the audio output device 30 are connected to each other via a predetermined interface. The acoustic signal in this embodiment includes either an analog signal or a digital signal, or both.

The signal processing device 10 and the related party terminal 70 may be connected to each other via a network (e.g., the Internet or an intranet) or may be connected to each other via a specified interface (e.g., a wireless interface using Bluetooth or the like). The signal processing device 10 and the related party terminal 70 may be connected via an external device (e.g., a server) not shown.

The signal processing device 10 performs audio signal processing on an input audio signal acquired from a sound source device 50. The acoustic signal processing by the signal processing device 10 includes at least modulation processing of the acoustic signal (to be described in detail later). Furthermore, the audio signal processing by the signal processing device 10 may include conversion processing of the audio signal (for example, separation, extraction, or synthesis). Furthermore, the audio signal processing by the signal processing device 10 may further include an amplification process of the audio signal similar to that of an AV amplifier, for example. The signal processing device 10 sends an output audio signal generated by the audio signal processing to the audio output device 30. The signal processing device 10 is an example of an information processing device.

The audio output device 30 generates a sound corresponding to the output sound signal obtained from the signal processing device 10. The audio output device 30 may include, for example, a loudspeaker (it may include a speaker with a built-in amplifier (a powered speaker)), headphones, earphones, or smart speakers.

The audio output device 30 can also be configured together with the signal processing device 10 as one device. Specifically, the signal processing device 10 and the audio output device 30 can be implemented in a TV, a radio, a music player, an AV amplifier, a speaker, headphones, earphones, a smartphone, a PC (including a tablet PC; the same applies below), or a smart speaker. The signal processing device 10 and the audio output device 30 constitute a cognitive function improvement system.

The sound source device 50 sends an input acoustic signal to the signal processing device 10. The sound source device 50 is, for example, a TV, a radio, a music player, a smartphone, a PC, an electronic musical instrument, a telephone, a game machine, an amusement machine, or a device that transmits an acoustic signal via broadcasting or information communication.

The related party terminal 70 can be implemented as a smartphone or a PC. The related party terminal 70 has a function of receiving information from the signal processing device 10 and presenting the notified information to the user of the related party terminal 70 (the related party).

In the following description, a related party is, for example, at least one of the following people:

- A user of the audio output device 30 (i.e., a person who listens to sounds to improve cognitive function, hereinafter referred to as a "patient")
- A family member, friend, or acquaintance of the patient
- Healthcare professionals (e.g., the patient's doctor, nurse, medical technician)
- Caregivers (e.g., the patient's designated caregiver)
- Creator or provider of content corresponding to the input audio signal (described later)
- Creator or provider of the content corresponding to the input audio signal
- Provider of the signal processing device 10
- Managers of facilities used by patients
- Insurance companies
- Other patients in the same community as the patient
- System provider
- Third parties designated by the parties mentioned above

The related party terminal 70 is possessed by the related party. "Possession" here is not limited to ownership, but can mean temporary appropriation for the purpose of operation. The related party terminal 70 may be shared by multiple related parties. Furthermore, the signal processing device 10 may notify information to a plurality of related party terminals 70.

(1-1) Configuration of the signal processing device

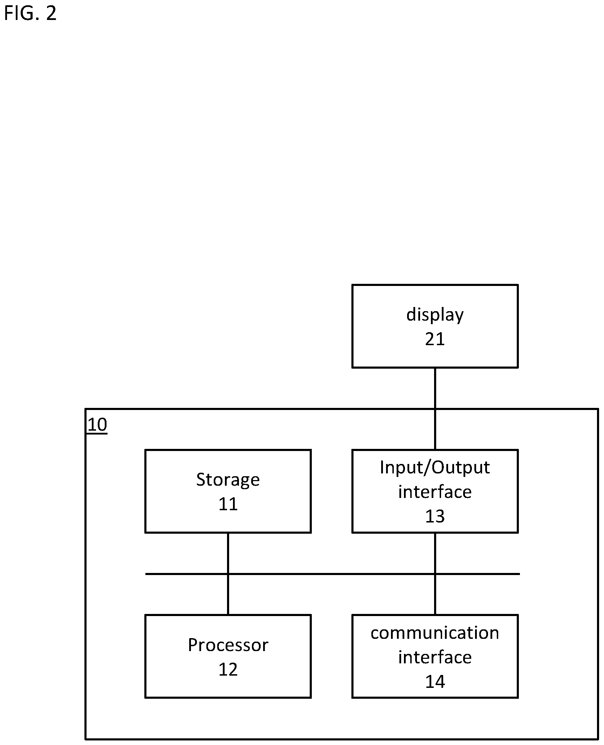

The configuration of the signal processing device will be described. FIG. 2 is a block diagram showing the configuration of a signal processing device according to this embodiment.

As shown in FIG. 2, the signal processing device 10 includes a storage 11, a processor 12, an input/output interface 13, and a communication interface 14. The signal processing device 10 is connected to a display 21.

The storage 11 is configured to store programs and data. The storage 11 is, for example, a combination of a Read Only Memory (ROM), a Random Access Memory (RAM), and a storage (for example, a flash memory or a hard disk). The program and data may be provided via a network, or may be provided by being recorded on a computer-readable recording medium.

The programs include, for example, the following programs:

- OS (Operating System) programs
- Application programs that perform information processing

The data includes, for example, the following data:

- Databases referenced in information processing

Data obtained by executing information processing (i.e., the results of executing information processing)

The processor 12 is a computer that realizes the functions of the signal processing device 10 by reading and executing the programs stored in the storage 11. At least a part of the functions of the signal processing device 10 may be realized by one or more dedicated circuits. The processor 12 may be, for example, at least one of the following:

- CPU (Central Processing Unit)
- GPU (Graphic Processing Unit)
- ASIC (Application Specific Integrated Circuit)
- FPGA (Field Programmable Array)
- DSP (Digital Signal Processor)

The input/output interface 13 is configured to obtain information (e.g., user instructions) from an input device connected to the signal processing device 10, and to output information (e.g., images or control signals) to an output device connected to the signal processing device 10.

The input device is, for example, a sound source device 50, a physical button, a keyboard, a pointing device, a touch panel, or a combination thereof.

The output device is, for example, the display 21, the audio output device 30, a light-emitting element, a speaker different from the audio output device 30, or a combination of these.

Furthermore, the input/output interface 13 may include signal processing hardware such as, for example, an A/D converter, a D/A converter, an amplifier, a mixer, a filter, and the like.

The communication interface 14 is configured to control communication between the signal processing device 10 and an external device (for example, the audio output device 30, the sound source device 50, or the related party terminal 70).

The display 21 is configured to display an image (still image or video image). The display 21 is, for example, a liquid crystal display or an organic EL display. Alternatively, the display 21 may be a seven-segment display.

(1-2) Configuration of related party terminals

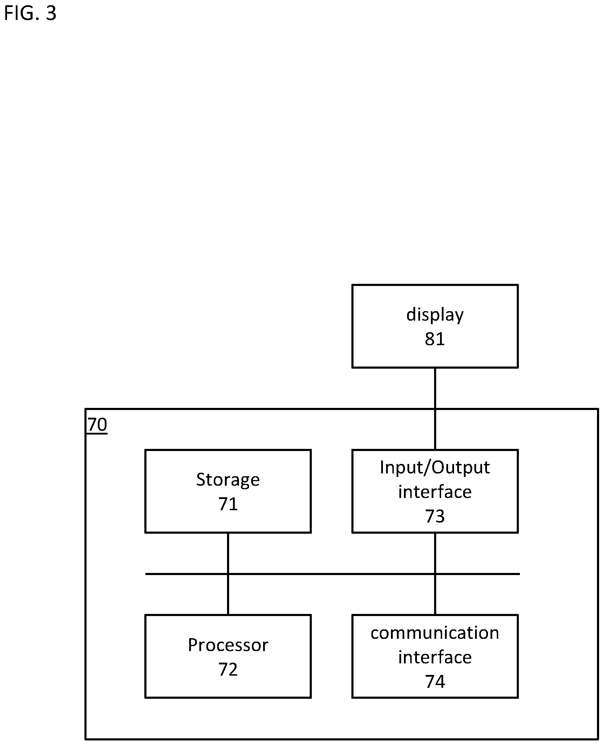

The configuration of the related party terminal will be described. FIG. 3 is a block diagram showing the configuration of the related party terminal of this embodiment.

As shown in FIG. 3, the related party terminal 70 includes a storage 71, a processor 72, an input/output interface 73, and a communication interface 74. The related party terminal 70 is connected to a display 81.

The storage 71 is configured to store programs and data. The storage 71 is, for example, a combination of ROM, RAM, and storage (for example, flash memory or a hard disk). The program and data may be provided via a network, or may be provided by being recorded on a computer-readable recording medium.

The programs include, for example, the following programs:

- OS program
- Application programs that perform information processing

The data includes, for example, the following data:

- Databases referenced in information processing
- Results of information processing

The processor 72 is a computer that realizes the functions of the related party terminal 70 by reading and executing the programs stored in the storage 71. At least a part of the functions of the related party terminal 70 may be realized by one or more dedicated circuits. The processor 72 may be, for example, at least one of the following:

- CPU
- GPU
- ASIC
- FPGA
- DSP

The input/output interface 73 is configured to obtain information (such as a user's instruction) from an input device connected to the related party terminal 70, and to output information (such as an image) to an output device connected to the related party terminal 70.

The input device is, for example, a physical button, a keyboard, a pointing device, a touch panel, or a combination thereof.

The output device is, for example, a display 81, a speaker, or a combination thereof.

The communication interface 74 is configured to control communication between the related party terminal 70 and an external device (e.g., the signal processing device 10).

The display 81 is configured to display an image (still image or video). The display 81 is, for example, a liquid crystal display or an organic EL display.

(2) One aspect of the embodiment

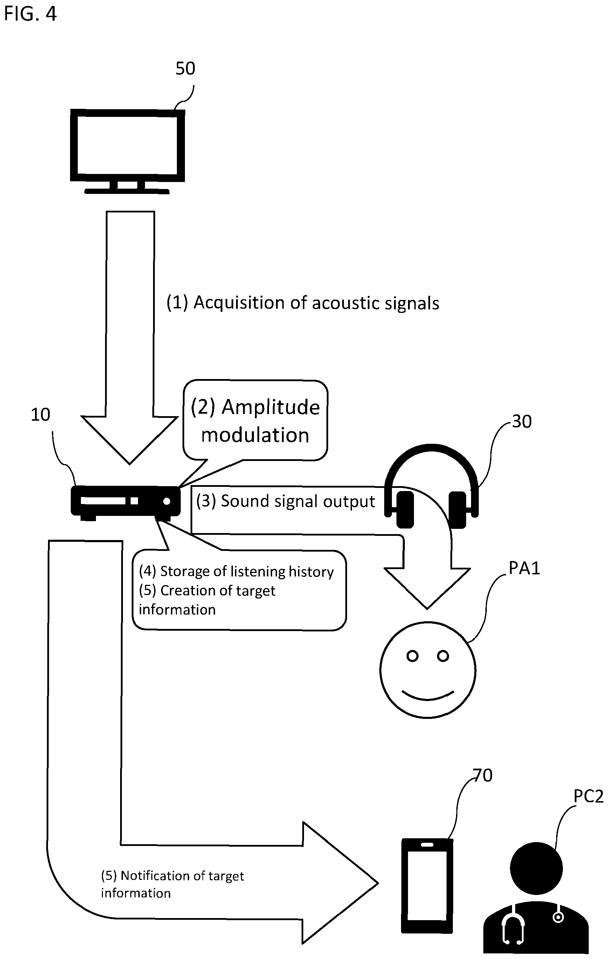

One aspect of this embodiment will be described. FIG. 4 is an explanatory diagram of one aspect of this embodiment.

As shown in FIG. 4, the signal processing device 10 obtains an acoustic signal (input acoustic signal) from a sound source device 50. The signal processing device 10 performs amplitude modulation processing on the acoustic signal. The signal processing device 10 causes the generated audio signal (output audio signal) to be output by the audio output device 30. Since the output acoustic signal has amplitude changes corresponding to the frequency of gamma waves, when patient PA1 hears the sound produced by the output acoustic signal, gamma waves are induced in the brain of patient PA1 (brain waves are synchronized to the gamma frequency). This is expected to have the effect of improving cognitive function (e.g., treating or preventing dementia). On the other hand, the signal processing device 10 stores the patient PA1's listening history (an example of "information on the output acoustic signal") for sounds for improving cognitive function (sounds emitted by the audio output device 30 in response to the output acoustic signal). As an example, the signal processing device 10 stores information regarding what type of amplitude modulation was applied to the acoustic signal having what features to generate the output acoustic signal, as well as at what playback volume and for how long the patient PA1 listened to the sound from the output acoustic signal, in a listening history database described later.

The signal processing device 10 generates information (hereinafter referred to as "target information") regarding the patient PA1's listening status for sounds for improving cognitive function based on the stored listening history, and notifies the related party terminal 70 of the target information. The related party terminal 70 presents the target information to the related party PC2. This allows the related party PC2 to understand the state in which the patient PA1 is hearing the sound.

This allows the related party PC2 to obtain various effects according to the attributes of the related party PC2.

For example, a family member or medical professional can encourage the patient PA1 to improve the patient's hearing condition based on the notified target information.

Based on the notified target information, medical professionals can confirm whether patient PA1 is acting in accordance with the treatment plan, create or modify a treatment plan regarding patient PA1's cognitive function based on the listening situation, and make a diagnosis regarding patient PA1's cognitive function based on the listening situation.

The patient PA1 can introspect on his/her own hearing condition based on the notified target information.

The facility manager can use the notified target information to efficiently manage the patient PA1 as a facility user.

The insurance company can use the notified target information to calculate a reasonable insurance premium for patient PA1.

The provider of the signal processing device 10 can use the notified target information to conduct research and development to improve the effect of the signal processing device 10 in improving cognitive function (e.g., more effective methods of generating and providing output acoustic signals, etc.). The target information notified to the related party PC2 may be anonymized. Also, the patient may be allowed to freely choose whether or not to provide the target information in exchange for a compensation (e.g., a coupon).

The creator or provider of the content corresponding to the input audio signal can obtain suggestions regarding guidelines for creating or providing the content from the notified target information. The target information notified to the related party PC2 may be anonymized. Also, the patient may be allowed to freely choose whether or not to provide the target information in exchange for a compensation (e.g., a coupon).

Other patients belonging to the same community can view the notified information to check the behavioral records of patients who are their rivals or peers and reflect on their own behavioral records, making it easier for them to maintain their motivation to continue listening to sounds to improve dementia. In addition, patients will be aware that their behavioral achievements are being seen by other patients in the same community, making it easier for them to maintain motivation to continue listening to sounds to improve their dementia.

(3) Database

The database of this embodiment will be described. The following databases are stored in the storage 11: Alternatively, the database may be stored in a storage device provided in an external device (e.g., a server) accessible by the signal processing device 10. In this case, information relating to a plurality of different signal processing devices 10 may be collected in one database.

(3-1) Listening history database

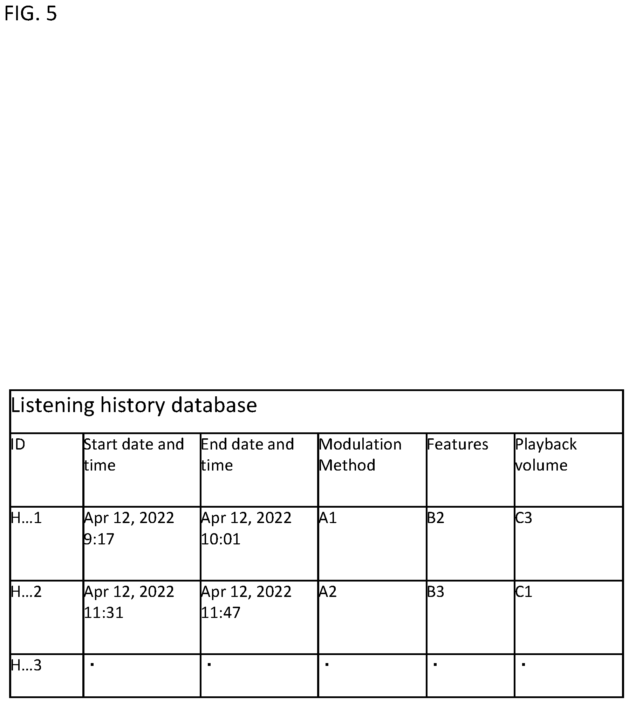

The listening history database of this embodiment will be described. FIG. 5 is a diagram showing the data structure of the listening history database of this embodiment.

The listening history database stores the listening history. The listening history is log information regarding the patient's listening behavior to sounds for improving cognitive function.

As shown in FIG. 5, the listening history database includes an "ID" field, a "start date and time" field, an "end date and time" field, a "modulation method" field, a "features" field, and a "playback volume" field. Each field is associated with another.

The "ID" field stores the listening history ID. The listening history ID is information for identifying the listening history.

The "start date and time" field stores start date and time information. The start date and time information is information about the date and time when the reproduction of the output acoustic signal started in the corresponding listening history.

The "end date and time" field stores end date and time information. The end date and time information is information about the date and time when the reproduction of the output acoustic signal ended in the corresponding listening history. That is, the period during which the patient listened to the sound of the output acoustic signal can be identified based on the start date and time information and the end date and time information.

The "modulation method" field stores modulation method information. The modulation method information is information about the processing performed to generate an output acoustic signal from an input acoustic signal in the corresponding listening history. For example, the modulation method information may include information regarding at least one of the following:

- Modulation function used for amplitude modulation
- Modulation depth
- Amplitude modulated audio signal
- Audio signal with no amplitude modulation applied

Here, when an intermediate acoustic signal (described in detail later) including multiple acoustic signals is generated based on an input acoustic signal, and a decision is made individually as to whether or not to apply amplitude modulation to the multiple acoustic signals, each of the multiple acoustic signals corresponds to either an "acoustic signal to which amplitude modulation has been applied" or an "acoustic signal to which amplitude modulation has not been applied." Similarly, when the input acoustic signal consists of multiple acoustic signals and a decision is made individually as to whether or not to apply amplitude modulation to each of the multiple acoustic signals, each of the multiple acoustic signals corresponds to either an "acoustic signal with amplitude modulation applied" or an "acoustic signal without amplitude modulation applied." In these cases, since the function used for amplitude modulation or the modulation depth may be different for each of the acoustic signals to which amplitude modulation is applied, the modulation method information may include information regarding the "function used for amplitude modulation" or "modulation depth" of each acoustic signal.

The "features" field stores feature information. The feature information is information on the feature of a sound signal to which amplitude modulation has been applied in the corresponding listening history. For example, the feature information may include information regarding at least one of the following:

- Amplitude, energy or rms distribution in the frequency domain (e.g., the result of a Fast Fourier Transform (FFT))
- Distribution of amplitude, energy or effective value in the time domain
- Sound source type (e.g., vocal, instrument, object, music, dialogue, natural sound, electronic sound, etc.)
- Content attributes (e.g., program information of a TV program that includes an audio signal)

The "playback volume" field stores playback volume information. The playback volume information is information related to the playback volume of the output audio signal in the corresponding listening history. The signal processing device 10 may control the playback volume information by specifying a playback volume for the audio output device 30, or may obtain the playback volume information from the audio output device 30.

(4) Information processing

The information processing of this embodiment will be described.

(4-1) Acoustic signal processing

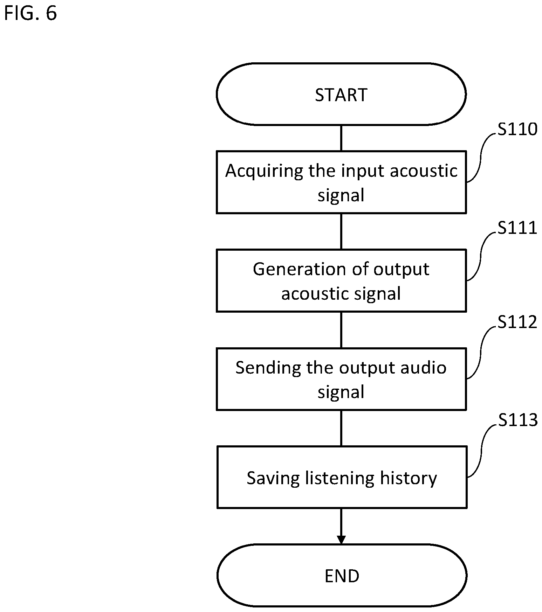

The acoustic signal processing of this embodiment will now be described. FIG. 6 is a flowchart of the acoustic signal processing according to this embodiment.

The acoustic signal processing in FIG. 6 is realized by the processor 12 of the signal processing device 10 reading and executing a program stored in the storage 11.

The acoustic signal processing in FIG. 6 starts when any of the following start conditions is met:

- The acoustic signal processing in FIG. 6 is called by another process or an external instruction;
- The related party performed an operation to call up the acoustic signal processing of FIG. 6;
- The signal processing device 10 is in a predetermined state (for example, power is turned on);
- The specified date and time has arrive;.
- A predetermined time has elapsed since a predetermined event (for example, the start-up of the signal processing device 10 or the previous execution of the acoustic signal processing in FIG. 6).

As shown in FIG. 6, the signal processing device 10 acquires an input sound signal (S110). Specifically, the signal processing device 10 receives an input acoustic signal sent from a sound source device 50.

In step S110, the signal processing device 10 may further perform A/D conversion of the input acoustic signal.

The input acoustic signal may correspond, for example, to at least one of the following:

- Musical content (e.g., singing, playing, or a combination thereof (i.e., a song). It may include audio content that accompanies the video content. )
- Audio content (e.g., readings, narrations, announcements, radio plays, solo performances, conversations, monologues, or combinations thereof. This may include audio content accompanying video content.)
- Other audio content (e.g., electronic, environmental, or mechanical sounds)

However, singing, or audio content, is not limited to sounds produced by the human vocal tract, but may also include sounds generated by voice synthesis technology.

After step S110, the signal processing device 10 generates an output acoustic signal (S111). Specifically, the signal processing device 10 generates an output acoustic signal by performing amplitude modulation on at least a part of the input acoustic signal acquired in step S110.

As a first example of generating an output acoustic signal (S111), the signal processing device 10 generates an intermediate acoustic signal including a plurality of acoustic signals having different characteristics based on an input acoustic signal. Here, the characteristics may be determined based on an input operation by a related party or an instruction from outside, or may be determined by an algorithm. For example, the signal processing device 10 may determine characteristics for generating an intermediate audio signal based on a result of analyzing an input audio signal.

The characteristics may be, for example, at least one of the following:

- Sound characteristics (especially qualitative characteristics)
- Frequency characteristics
- Time characteristics
- Amplitude characteristics
- Output characteristics

The signal processing device 10 selects one or more acoustic signals (hereinafter referred to as "target signals") to which amplitude modulation is to be applied from the multiple acoustic signals included in the intermediate acoustic signal. Which acoustic signal is selected as the target signal may be determined based on an input operation by a related party or an external instruction, or may be determined by an algorithm. For example, the signal processing device 10 may determine the target signal based on characteristics of the audio signal (balance between speech and music, volume change, type of music, timbre, or other characteristics). This enables the signal processing device 10 to select a target signal that will have a greater effect on improving cognitive function through modulation, or to select a target signal that will cause the patient less discomfort. However, the signal processing device 10 may treat all of the multiple acoustic signals included in the intermediate acoustic signal as target signals.

The signal processing device 10 performs amplitude modulation on the selected target signal. As an example, the signal processing device 10 performs amplitude modulation on the target signal using a modulation function having a frequency corresponding to gamma waves (for example, a frequency not less than 35 Hz and not more than 45 Hz). Specifically, assuming that a modulation function having a periodicity of 35 Hz or more and 45 Hz or less is A(t), a function representing the waveform of the acoustic signal before modulation is X(t), and a function representing the waveform of the modulated acoustic signal is Y(t),

$$Y(t) = A(t)\, X(t)$$

It becomes. As a result, a change in amplitude corresponding to the frequency is added to the target signal. Here, the signal processing device 10 may determine a modulation function used for the amplitude modulation of the target signal or a modulation depth of the amplitude modulation. The modulation function or modulation depth may be determined based on an input operation by a related party or an external instruction, or may be determined by an algorithm. Furthermore, the signal processing device 10 may determine a common modulation function or modulation depth for a plurality of target signals, or may determine a modulation function or modulation depth for each of a plurality of target signals.

The signal processing device 10 generates an output acoustic signal based on acoustic signals that are not selected as target signals (hereinafter referred to as "non-target signals") from among the multiple acoustic signals included in the intermediate acoustic signal, and the modulated target signal. That is, the signal processing device 10 converts the non-target signal and the modulated target signal into an output acoustic signal. Specifically, the signal processing device 10 combines two or more acoustic signals among the non-target signal and the modulated target signal, or extracts or separates an acoustic signal from at least one of the non-target signal and the modulated target signal. The method of synthesizing the acoustic signals is not limited, but may include, for example, signal summation processing, HRTF (Head Related Transfer Function) convolution processing, convolution processing of a transfer function that provides position information of the sound source, or summation processing after performing these convolution processes. Furthermore, the signal processing device 10 may further perform at least one of amplification, volume adjustment, and D/A conversion of the output acoustic signal. On the other hand, if the asymmetric signal and the modulated target signal match the output format of the audio output device 30 (for example, if the asymmetric signal and the modulated target signal correspond to multi-channel audio signals associated with each speaker that constitutes a surround system as the audio output device 30), such conversion is not necessary. In this case, the non-target signal and the modulated target signal are treated as the output acoustic signal.

In a second example of generating an output acoustic signal (S111), the input acoustic signal includes a plurality of acoustic signals with different characteristics. The signal processing device 10 selects one or more acoustic signals to which amplitude modulation is to be applied from a plurality of acoustic signals included in an input acoustic signal. The second example of the generation of the output acoustic signal (S111) can be understood by appropriately replacing the "intermediate acoustic signal" with the "input acoustic signal" in the explanation of the first example above.

In the third example of generating an output acoustic signal (S111), the signal processing device 10 performs amplitude modulation on the input acoustic signal. The amplitude modulation of the input acoustic signal is similar to the amplitude modulation of the target signal described in the first example of the generation of the output acoustic signal (S111). As a result, a change in amplitude corresponding to the frequency of gamma waves is added to the input acoustic signal. Here, the signal processing device 10 may determine a modulation function used for the amplitude modulation of the input acoustic signal or a modulation depth of the amplitude modulation. The modulation function or modulation depth may be determined based on an input operation by a related party or an external instruction, or may be determined by an algorithm.

The signal processing device 10 generates an output audio signal based on the modulated input audio signal. That is, the signal processing device 10 converts a modulated input audio signal into an output audio signal.

Specifically, when the modulated input acoustic signal is made up of multiple acoustic signals, the signal processing device 10 synthesizes two or more of the multiple acoustic signals, or extracts or separates an acoustic signal from the modulated input acoustic signal. The details of the method for synthesizing the acoustic signals are as explained in the first example of the generation of the output acoustic signal (S111). On the other hand, if the modulated input audio signal matches the output format of the audio output device 30 (for example, if the modulated input audio signal corresponds to a multi-channel audio signal associated with each speaker that constitutes a surround system as the audio output device 30), such conversion is not necessary. In this case, the modulated input audio signal is treated as the output audio signal.

After step S111, the signal processing device 10 executes transmission of an output acoustic signal (S112).

Specifically, the signal processing device 10 sends the output acoustic signal generated in step S111 to the audio output device 30. The audio output device 30 generates a sound according to the output sound signal.

After step S112, the signal processing device 10 executes storage of the listening history (S113). Specifically, the signal processing device 10 stores a listening history including at least one of the following in a listening history database (FIG. 5):

- Information regarding the feature of the audio signal to which amplitude modulation has been applied in step S111
- Information regarding the modulation method of the amplitude modulation performed in step S111
- Information regarding the date and time when the audio output device 30 started playing the output sound signal sent in step S112

- Information regarding the playback volume set in the audio output device 30 when the output audio signal sent in step S112 was played back.
- Information regarding the date and time when the audio output device 30 finished reproducing the output sound signal sent in step S112

After step S113, the signal processing device 10 ends the acoustic signal processing in FIG. 6.

The signal processing device 10 may perform the audio signal processing of FIG. 6 all at once on an input audio signal having a fixed playback period (e.g., one piece of music content), or may repeatedly perform the audio signal processing of FIG. 6 for each predetermined playback section of the input audio signal (e.g., every 100 ms). Alternatively, the signal processing device 10 may continuously perform modulation processing on the input acoustic signal, such as modulation by analog signal processing, and output a modulated acoustic signal. The acoustic signal processing shown in FIG. 6 may be terminated in response to a specific termination condition (e.g., a certain amount of time has elapsed, an operation has been performed by a related party, or the output history of modulated sound has reached a predetermined state).

(4-2) Information notification processing

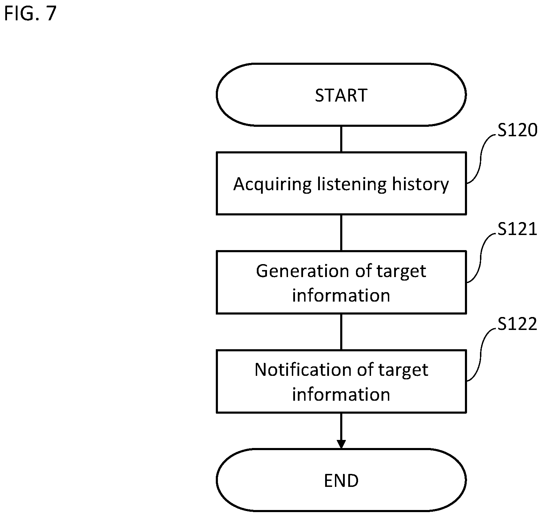

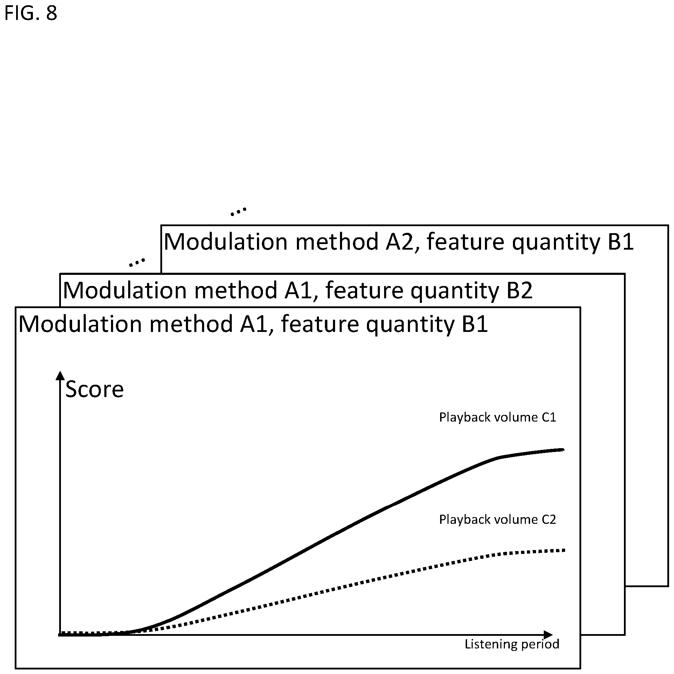

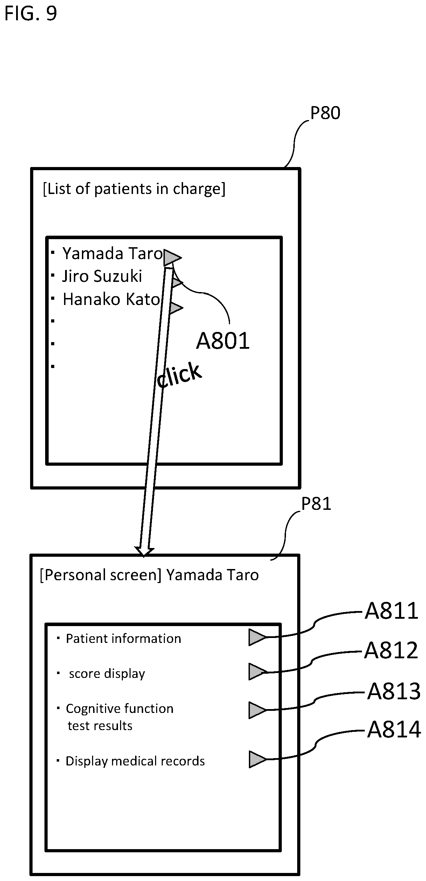

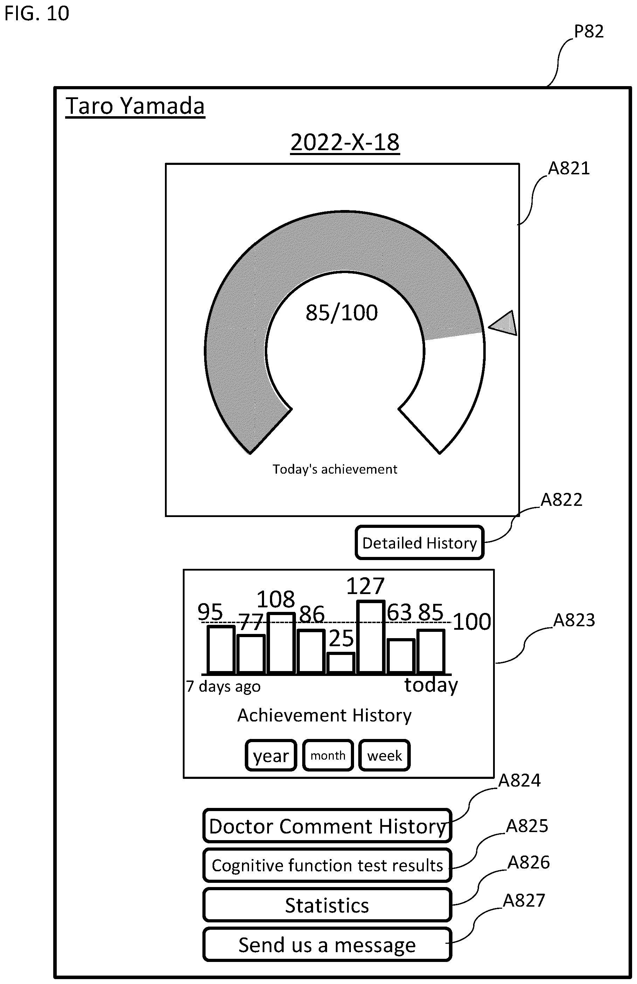

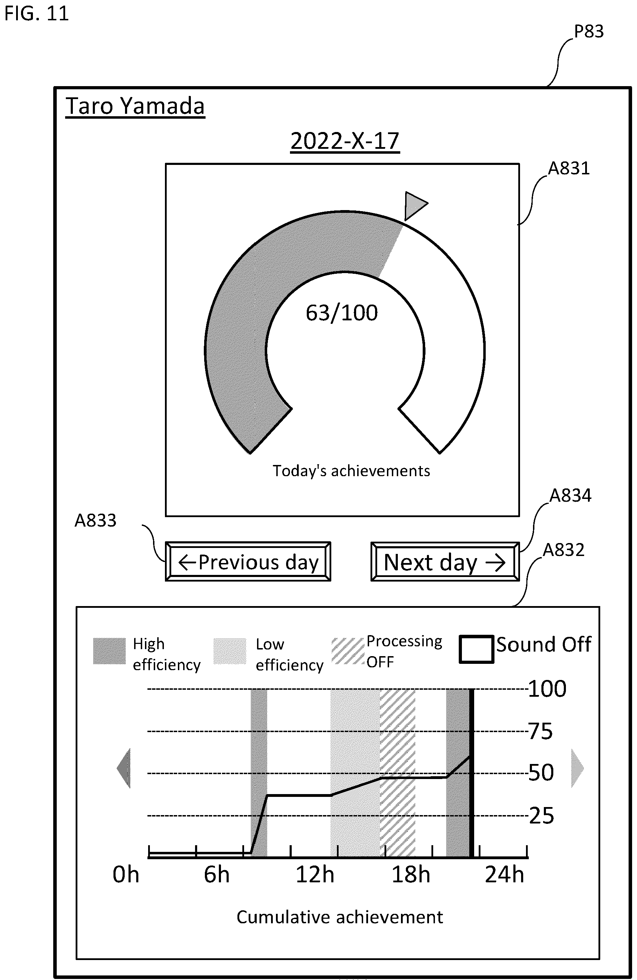

The information notification process of this embodiment will be described. FIG. 7 is a flowchart of the information notification process according to this embodiment. FIG. 8 is a diagram showing an example of a score function used in the information notification process of this embodiment. FIG. 9 is a diagram showing an example of presentation of target information in the information notification process of this embodiment. FIG. 10 is a diagram showing an example of presentation of target information in the information notification process of this embodiment. FIG. 11 is a diagram showing an example of presentation of target information in the information notification process of this embodiment.

The information notification process in FIG. 7 starts when any one of the following start conditions is met.

- The information notification process in FIG. 7 is called by another process or an external instruction.
- The related party performs an operation to call up the information notification process shown in FIG. 7.
- The signal processing device 10 is in a predetermined state (for example, power is turned on).
- The specified date and time has arrived.
- A predetermined time has elapsed since a predetermined event (for example, start-up of the signal processing device 10, saving of a new listening history, or previous execution of the information notification process in FIG. 7).

As shown in FIG. 7, the signal processing device 10 executes acquisition of a listening history (S120). Specifically, the signal processing device 10 acquires the patient's hearing history from the hearing history database (FIG. 5). As an example, the signal processing device 10 extracts, from the listening history database, listening histories in which at least one of the start date and time information or the end date and time information belongs to the period for which the target information is generated (hereinafter referred to as the "target period"). Here, the target period may be any of the following:

- The day, week, or month to which the information notification process of FIG. 7 was executed
- A predetermined period going back from when the information notification process of FIG. 7 was executed
- For a period specified by the related parties
- A period specified according to an algorithm

After step S120, the signal processing device 10 generates target information (S121).

Specifically, the signal processing device 10 generates target information based on the listening history acquired in step S120. The target information may include, for example, at least one of the following:

- Listening history (e.g., the period during which the patient listened to sounds for cognitive improvement)
- Information that has been processed from listening history (e.g., statistical information)
- A score on the extent of improvement in cognitive function according to the sounds the patient heard

Regarding the calculation of the score, as an example, the signal processing device 10 can refer to a score function to derive a score corresponding to elements included in the listening history (e.g., the listening period of the sound, the playback volume of the output audio signal, the modulation method of the amplitude modulation applied to the audio signal, the features of the audio signal to which the amplitude modulation has been applied, or a combination thereof). The score function may be saved in the storage 11, or may be stored in a storage device provided in an external device (e.g., a server) accessible by the signal processing device 10.

Here, the score represents the degree of improvement in cognitive function, and is defined to correlate with the estimated results of the amount of EEG elicitation by sound stimulation (i.e., the extent to which gamma waves were induced), an index calculated from the degree of EEG synchronization at each time (i.e., the duration for which gamma wave synchronization was achieved), or an index calculated from the EEG elicitation ratio at each time (i.e., the history of the extent to which gamma waves were induced compared to other EEG bands). As an example, the score is determined to increase (e.g., increase linearly) with increasing amount of electroencephalography evoked. As another example, the score is determined so that it hardly increases when the amount of brain wave evoked is below the first threshold, and increases in response to an increase in the amount of brain wave evoked after the amount of brain wave evoked exceeds the first threshold. As yet another example, the score is determined so that it increases in response to an increase in the amount of brainwave evoked energy when the amount of brainwave evoked energy is below the second threshold, and so that it increases very little (i.e., saturates) once the amount of brainwave evoked energy exceeds the second threshold. By having a subject listen to various acoustic signals under various conditions and measuring the brain wave elicitation of the subject using an electroencephalograph, it is possible to describe the relationship between the elements contained in the listening history and the brain wave elicitation. A score function for deriving a score corresponding to an element included in the listening history can be created based on the relationship between the element included in the listening history and the amount of electroencephalogram elicitation, and the relationship between the score and the amount of electroencephalogram elicitation. The score function may be defined as a mathematical expression that takes the elements of the listening history as arguments, or may be defined as a lookup table.

As an example, the signal processing device 10 calculates a score corresponding to the listening history using a score function shown in FIG. 8. In the score function of FIG. 8, the score is defined to increase or maintain (in other words, not decrease) with increasing listening period. Moreover, the score function in FIG. 8 is defined so that the score for a playback volume C1 is higher than the score for a playback volume C2 (lower than C1).

When the signal processing device 10 acquires multiple listening histories in step S120, the signal processing device 10 may calculate the change in the cumulative score during the target period (i.e., the change in the cumulative score over time) by cumulatively adding up the scores corresponding to each listening history.

In a case where a target score for the patient in the target period is set, the signal processing device 10 may calculate the ratio of the cumulative score to the target score as the patient's degree of achievement. The target score corresponds to the standard score (i.e., the sounds that should be heard) that the patient should achieve within the target period in order to improve cognitive function. The signal processing device 10 may calculate the degree of achievement at a single point in time, or may calculate the change in the degree of achievement over a target period (i.e., the change in the degree of achievement over time).

The signal processing device 10 may store the score calculation results in a database (not shown). This makes it possible to obtain previously calculated scores in a short time and with minimal consumption of computational resources.

After step S121, the signal processing device 10 executes notification of the target information (S122). Specifically, the signal processing device 10 transmits information necessary for presenting the target information generated in step S121 (e.g., the target information itself, or information regarding light, images, sounds, characters, movements of equipment, etc. that can be used to present the target information) to the related party terminal 70.

Information regarding the notification destination of the target information (hereinafter referred to as "notification destination information") is defined in association with information for identifying a patient (for example, a patient ID). The notification destination information is the account name, email address, or telephone number of the related party to be notified. By using the notification destination information, it is possible to identify the related party terminal 70 to which the information required for presenting the target information is to be transmitted.

The signal processing device 10 can notify the target information in at least one of the following formats, for example:

- Application notifications
- SNS (Social Networking Service) messages
- Email
- SMS (Short Service Message) messages
- Information that can be viewed after logging in to a specific application or SaaS (Software as a Service) (e.g., messages)

The related party terminal 70 presents the target information to the related party via an output device. The signal processing device 10 can express the target information using at least one of light, sound, text, an image, and the movement of a device or the like.

As mentioned above, the subject information may include a score. The score included in the target information may be at least one of the following:

- Score for each listening history
- Cumulative score at any point during the target period
- Changes in cumulative scores during the target period
- Degree of achievement at any point during the target period
- Changes in achievement levels during the target period
- Degree of failure to reach the target score

As an example, as shown in FIGS. 9, 10 and 11, the related party terminal 70 can present the target information to the related party by an image displayed on the display 81.

When the related party has the authority to view the target information of multiple patients (typically when the related party corresponds to a medical professional or a care professional, etc.), the related party terminal 70 displays screen P80 of FIG. 9 on the display 81. On the other hand, when the related party only has the authority to view the target information of one patient (typically when the related party corresponds to the patient himself/herself or a family member of the patient), the related party terminal 70 displays screen P81 of FIG. 9 on the display 81.

The screen P80 displays a list of patients whose target information can be viewed. As shown in FIG. 9, a screen P80 includes an object A801.

Object A801 accepts a user instruction that specifies which patient's target information is to be viewed. When object A801 is selected (or its name is clicked), the related party terminal 70 displays screen P81 on the display 81.

The screen P81 is a menu screen for selecting which information on the patient to view. As shown in FIG. 9, a screen P81 includes objects A811 to A812. Depending on the authority of the related party (for example, whether or not he/she is a doctor), the selectable objects or the display contents on the menu screen may be restricted.

Object A811 accepts a user instruction to display basic information about a patient. When object A811 is selected, the related party terminal 70 displays on the display 81 a screen (not shown) displaying basic information about the patient.

Object A812 accepts user instructions to display the patient's scores, etc. When object A812 is selected, the related party terminal 70 displays a screen P82 shown in FIG. 10.

Object A813 accepts user instructions to display the results of a patient's cognitive function test. When object A811 is selected, the related party terminal 70 displays on the display 81 a screen (not shown) that displays the results of the patient's cognitive function test.

Object A814 accepts a user instruction to display a patient's chart. When object A814 is selected, the related party terminal 70 displays on the display 81 a screen (not shown) showing the results of the patient's medical record.

The screen P82 is a dashboard screen regarding the patient's listening status. As shown in FIG. 10, a screen P82 includes objects A821 to A827. Depending on the authority of the related party (for example, whether or not he/she is a doctor), selectable objects or display contents may be limited on the dashboard screen.

Object A821 displays the patient's achievement level for today (i.e., the day screen P82 is viewed). Object A821 represents the patient's achievement level using a pie chart and numbers.

Object A822 accepts a user instruction to display a detailed listening history for a patient. When object A822 is selected, the related party terminal 70 displays on the display 81 a screen (not shown) showing the detailed interview history of the patient.

Object A823 displays the patient's past achievement history using a bar graph. The achievement history displayed in object A823 can be switched between yearly, monthly, and weekly units. When a bar representing the degree of achievement of any one of the days is selected from among the object A823, the related party terminal 70 displays the screen P83 of FIG. 11.

Object A824 accepts a user instruction to display the history of physician comments. When object A824 is selected, the related party terminal 70 displays on the display 81 a screen (not shown) that displays the history of doctor comments.

Object A825 accepts user instructions to display the results of a patient's cognitive function test. When object A825 is selected, the related party terminal 70 displays on the display 81 a screen (not shown) that displays the results of the patient's cognitive function test.

Object A826 accepts user instructions for displaying statistical information. When object A826 is selected, the related party terminal 70 displays a screen (not shown) displaying statistical information on the display 81. Here, the statistical information corresponds to the result of statistical processing of the listening histories or scores collected for a plurality of patients. The listening histories or scores of multiple patients can be collected by an external device (e.g., a server not shown) from the signal processing device 10 and other signal processing devices having similar functions to the signal processing device 10. The related party terminal 70 may display statistical information in comparison with the patient's listening history or score.

Object A827 accepts a user instruction to display an editing screen for a message to be sent. When object A827 is selected, the related party terminal 70 displays an editing screen (not shown) for a message to be sent on the display 81.

Screen P83 is a screen that displays the patient interview status on a specific date selected by the related party. As shown in FIG. 11, a screen P83 includes objects A831 to A834.

Object A831 displays the patient's achievements for the particular date selected. Object A831 represents the patient's achievement level using a pie chart and numbers.

Object A832 uses a line graph to represent the progress of the patient's achievement on a selected specific date. In object A832, periods when the rate of increase in achievement per unit time was high ("high efficiency"), periods when the rate of increase in achievement per unit time was low ("low efficiency"), periods when processing of the input audio signal (amplitude modulation) was disabled ("processing OFF"), and periods when playback of the output audio signal was stopped ("audio OFF") are clearly shown on the time axis. The efficiency may be classified into three or more stages instead of two stages. The current time may also be displayed.

Object A833 accepts a user instruction to shift the selected date forward by one day. When object A833 is selected, the related party terminal 70 updates the contents of objects A831 to A832.

Object A834 accepts a user instruction to shift the selected date forward by one day. When object A834 is selected, the related party terminal 70 updates the contents of objects A831 to A832.

9 to 11 show examples in which target information regarding the listening situation of a selected patient is displayed. However, the present invention is not limited to this. The signal processing device 10 may transmit the target information related to a plurality of patients to the related party terminal 70, and the related party terminal 70 may display the target information related to the plurality of patients simultaneously. For example, the related party terminal 70 may display the information on the screen P82 relating to each of a plurality of patients side by side on the display 81. Furthermore, the number of information items, display size, or amount of information displayed on the display 81 may be changed depending on the number of patients for which information is to be displayed. This allows for efficient display of target information for multiple patients on one screen.

The target information may be presented on an app screen or a website screen, or may be presented using a viewing screen for emails or other messages (e.g., SNS messages, SMS messages, etc.). Alternatively, the target information may be displayed on a screen of a smart speaker, television, smart watch, car navigation system, etc.

Alternatively, the related party terminal 70 may represent the score by a sound output from a speaker (one example of an output device).

After step S122, the signal processing device 10 ends the information notification process in FIG.

(5) Summary

As described above, the signal processing device 10 of this embodiment outputs an acoustic signal (i.e., an output acoustic signal) that is generated by amplitude modulation and has an amplitude change corresponding to the frequency of gamma waves, and also stores information about the output acoustic signal (e.g., listening history). The signal processing device 10 transmits a notification based on the stored information to a device external to the signal processing device 10 (i.e., the related party terminal 70). This allows related parties to understand the patient's hearing status for sounds intended to improve cognitive function.

The signal processing device 10 may send a notification to at least one of a device owned by the patient (i.e., the user of the audio output device 30), a device owned by the patient's family member, a device owned by the patient's caregiver, or a device owned by a medical professional. This allows at least one of the patient himself/herself, the patient's family, the patient's caregiver, and/or medical staff to understand the patient's hearing condition.

The information on the acoustic signal stored by the signal processing device 10 may include information regarding at least one of the period during which the acoustic signal was output, the modulation method applied to the acoustic signal, the loudness (volume) of the sound emitted in response to the acoustic signal, or features of the acoustic signal. This allows related parties to be notified of details of the patient's hearing status.

The notification may include information indicative of the listening history and/or a score determined based on the listening history. This allows related parties to understand the details of the patient's hearing situation.

The output acoustic signal may have amplitude variations corresponding to frequencies between 35 Hz and 45 Hz. This can induce gamma waves in the patient's brain, improving cognitive function.

(6) Modifications

A modification of this embodiment will now be described.

(6-1) Modification 1

Modification example 1 will be described. The first modification is an example in which the notification of target information is performed conditionally.

(6-1-1) Information notification processing

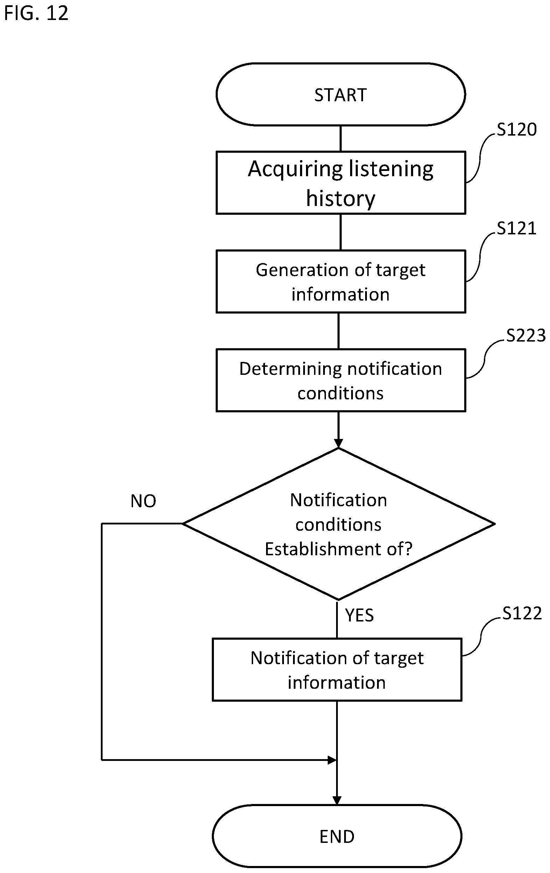

The information notification process of the first modification will be described. FIG. 12 is a flowchart of the information notification process of the first modified example.

The information notification process of the first modification starts when the same start condition as that of the information notification process of the present embodiment is satisfied.

As shown in FIG. 12, the signal processing device 10 executes acquisition of a listening history (S120) and generation of target information (S121). The details of obtaining the listening history (S120) and generating the target information (S121) are as described in the present embodiment.

After step S121, the signal processing device 10 executes a determination of the notification condition (S223).

Specifically, the signal processing device 10 acquires information corresponding to the determination material, and determines whether or not a predetermined notification condition is met based on the acquired information. The notification conditions may be editable by the related party. Notification conditions may be defined for each related party.

As a first example of the determination of the notification condition (S223), the signal processing device 10 may acquire a listening history collected about the patient, and determine whether or not the acquired listening history satisfies a predetermined notification condition. For example, if no listening history has been recorded for even one day in the last week, or if listening history has been recorded every day in the last three days, the signal processing device 10 may determine that the notification condition is met.

As a second example of the determination of the notification condition (S223), the signal processing device 10 may obtain a score calculated for the patient and determine whether or not the obtained score satisfies a predetermined notification condition. For example, when the average of the achievement level for each day in the most recent week is below or above a threshold value, the signal processing device 10 may determine that the notification condition is met.

As a third example of determining whether or not a notification condition is satisfied (S223), the signal processing device 10 may acquire the results of a cognitive function test for the patient, and determine whether or not the acquired results satisfy a predetermined notification condition. For example, if the results of a cognitive function test indicate that the patient's cognitive function class has changed (improved or worsened), the signal processing device 10 may determine that a notification condition is met. The signal processing device 10 may obtain the results of the cognitive function test from an external device, or may obtain the results by the signal processing device 10 itself administering the cognitive function test to the patient. In other words, the signal processing device 10 may be equipped with a function for conducting a cognitive function test (for example, a function for measuring cognitive function through voice dialogue with a patient).

If it is determined in step S223 that the notification condition is met, the signal processing device 10 executes notification of the target information (S122). The details of the notification of the target information (S122) are as described in the present embodiment.

After step S122, the signal processing device 10 ends the information notification process of FIG. Furthermore, if it is determined in step S223 that the notification condition is met, the signal processing device 10 ends the information notification process in FIG.

### (6-1-2) Summary

As described above, the signal processing device 10 of the first modification performs notification of the target information under certain conditions. For example, the signal processing device 10 may transmit a notification in response to the listening history or the score satisfying a predetermined condition. This allows timely feedback to the related parties on the patient's signs of abandoning listening to the sounds or on the patient's efforts, making it easier for the related parties to encourage the patient to continue listening to the sounds.

Alternatively, the signal processing device 10 may obtain the results of a cognitive function test of the patient, and transmit a notification when the results of the cognitive function test satisfy a predetermined condition. This makes it possible to use the results of cognitive function tests as an opportunity to encourage related parties to consider future actions for the patient (for example, setting goals regarding listening to sounds in order to improve cognitive function).

### (6-2) Modification 2

The second modification will be described. Modification 2 is an example in which target information is generated taking into account the patient's listening environment.

### (6-2-1) Sound system configuration

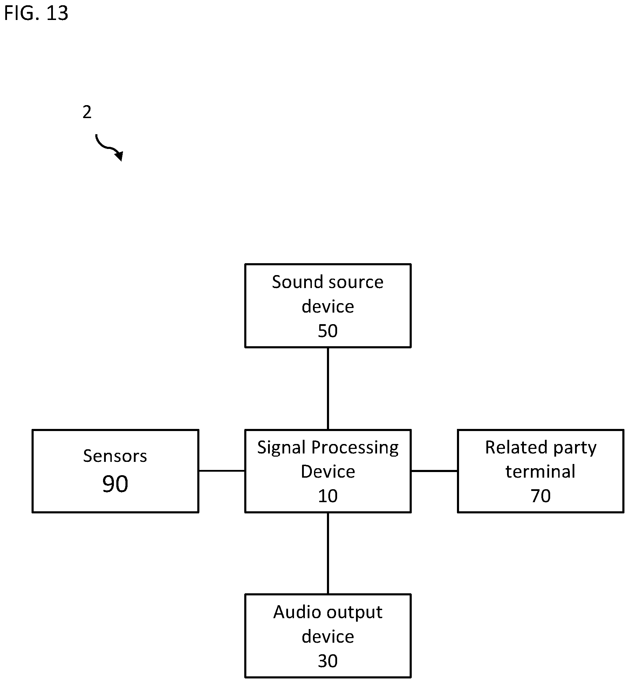

The configuration of the audio system will now be described. FIG. 13 is a block diagram showing the configuration of an audio system according to the second modification.

As shown in FIG. 13, the audio system 2 includes a signal processing device 10, an audio output device 30, a sound source device 50, a related party terminal 70 , and a sensor 90.

The signal processing device 10 and the sensor 90 may be connected to each other via a network (e.g., the Internet or an intranet) or via a predetermined interface (e.g., a wireless interface using Bluetooth or the like).

The sensor 90 performs sensing regarding the patient's listening environment. Specifically, the sensor 90 measures a physical quantity related to at least one of the following:

- The patient's listening position (particularly, the position relative to the audio output device 30)
- Sound pressure generated at the patient's listening position by the sound emitted from the audio output device 30
- The patient's behavior when listening to the sound emitted from the audio output device 30 (for example, whether the patient is concentrating on listening or is paying attention to something else)

The sensors 90 may include, for example, at least one of the following:

- Devices that constitute a radio wave positioning system (e.g., Bluetooth AoA (Angle of Arrival)) (e.g., devices that transmit or receive radio signals)
- Distance measurement sensor (e.g., ultrasonic sensor or LIDAR (Light Detection And Ranging))
- Pressure sensors embedded in, for example, chairs, beds, or floors
- Camera (visible light camera or infrared camera)
- Microphone

### (6-2-2) Information processing

The information processing of the second modification will be described.

### (6-2-2-1) Acoustic signal processing

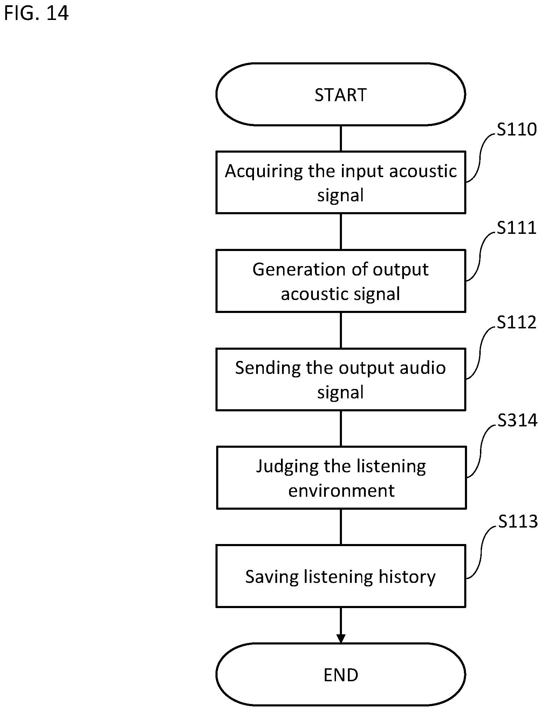

The acoustic signal processing according to the second modification will be described. FIG. 14 is a flowchart of the acoustic signal processing according to the second modification.

The acoustic signal processing of the second modification starts when the same start condition as that of the acoustic signal processing of this embodiment is satisfied.

As shown in FIG. 14, the signal processing device 10 acquires an input acoustic signal (S110), generates an output acoustic signal (S111), and transmits the output acoustic signal (S112). The details of obtaining an input acoustic signal (S110), generating an output acoustic signal (S111), and sending the output acoustic signal (S112) are as described in the present embodiment.

After step S212, the signal processing device 10 executes a determination of the listening environment (S214). Specifically, the signal processing device 10 acquires the sensing result of the sensor 90. The signal processing device 10 determines the listening environment of the patient based on the sensing result. The patient's listening environment may include, for example, at least one of the following:

- The distance from the audio output device 30 to the listening position of the patient, or the classification result thereof
- The direction from the audio output device 30 to the listening position of the patient, or the classification result thereof
- The sound pressure generated at the patient's listening position by the sound emitted from the audio output device 30 (i.e., the degree to which the sound emitted from the audio output device 30 is attenuated), and the classification result thereof
- The patient's behavior when listening to the sound emitted from the audio output device 30 (e.g., type of activity such as sitting, lying down, exercising, operating a smartphone, etc.)

After step S214, the signal processing device 10 executes storage of the listening history (S113).

Specifically, the signal processing device 10 stores a listening history including information on the determination result in step S214 (hereinafter referred to as "listening environment information") in a listening history database. Other elements that can be included in the listening history are as described in this embodiment.

After step S113, the signal processing device 10 ends the acoustic signal processing of FIG. 14.

The signal processing device 10 may perform the acoustic signal processing of FIG. 14 all at once on an input acoustic signal having a fixed playback period (e.g., one piece of music content), or may repeatedly perform the acoustic signal processing of FIG. 14 for each predetermined playback section of the input acoustic signal (e.g., every 100 ms). Alternatively, the signal processing device 10 may continuously perform modulation processing on the input acoustic signal, such as modulation by analog signal processing, and output a modulated acoustic signal. The acoustic signal processing shown in FIG. 14 may be terminated in response to a specific termination condition (e.g., a certain amount of time has elapsed, an operation has been performed by a related party, or the output history of modulated sound has reached a predetermined state).

(6-2-2-2) Information notification processing

The information notification process of the second modification will be described.

The information notification process of the second modification starts when the same start condition as that of the information notification process of the present embodiment is satisfied.

The signal processing device 10 executes acquisition of a listening history (S120). The details of obtaining the listening history (S120) are as described in the present embodiment.

After step S120, the signal processing device 10 generates target information (S121).

Specifically, the signal processing device 10 generates target information based on the listening history acquired in step S120. For example, the generated target information may include listening environment information or information obtained by processing the listening environment information (e.g., statistical information). Furthermore, similarly to the present embodiment, the signal processing device 10 may calculate a score corresponding to the listening history by using a score function. In the second modification, the signal processing device 10 may further correct the score corresponding to a listening history in accordance with listening environment information included in the listening history.

As a first example of generating target information (S121), the signal processing device 10 corrects the score so as to decrease or maintain the score with an increase in the distance from the audio output device 30 to the listening position of the patient. As an example, assuming that the score before correction is the same, the signal processing device 10 corrects the score so that when the distance is a first value, the score is smaller than when the distance is a second value (the second value is smaller than the first value). At listening positions far from the audio output device 30, there may be a decrease in the sound pressure reaching the patient and a relative decrease in the degree of modulation due to room reverberation or external sounds, which may reduce the effect of listening on improving cognitive function; however, by making such corrections, a more reasonable score can be obtained.

As a second example of generating target information (S121), the signal processing device 10 corrects the score to decrease or maintain the score in response to an increase in the difference between the direction from the audio output device 30 to the patient's listening position and the direction of the direction of the audio output device 30 (hereinafter referred to as the "directional difference"). As an example, assuming that the score before correction is the same, the signal processing device 10 corrects the score so that when the directional difference is a first value, the score is smaller than when the directional difference is a second value (the second value is smaller than the first value). The amount of correction may be determined according to the directional characteristics (gain characteristics) of the audio output device 30. If the listening position is in a direction away from the directional direction of the audio output device 30, the sound pressure reaching the patient or the modulation degree will decrease, which may reduce the effect of listening on improving cognitive function; however, by making such corrections, a more reasonable score can be obtained.

As a third example of generating target information (S121), the signal processing device 10 corrects the score to reduce or maintain the score in response to a decrease in sound pressure or a decrease in modulation level caused at the patient's listening position by the sound emitted from the audio output device 30. As an example, assuming that the score before correction is the same, the signal processing device 10 corrects the score so that when the sound pressure is a first value, the score is smaller than when the sound pressure is a second value (the second value is greater than the first value). If the sound pressure reaching the patient or the modulation level decreases, the effect of listening on improving cognitive function may decrease; however, by making such corrections, a more reasonable score can be obtained.

As a fourth example of generating target information (S121), a correction amount is predetermined for each patient's behavior (e.g., listening situation, state of alertness, or stress level) when listening to the sound emitted from the audio output device 30, and the signal processing device 10 corrects the score according to the correction amount. The effect of improving cognitive function as a result of listening to the sound emitted from the audio output device 30 may vary depending on the patient's behavior when listening to the sound. However, by making such corrections, a more appropriate score can be obtained.

After step S120, the signal processing device 10 executes notification of the target information (S122). The details of the notification of the target information (S122) are as described in the present embodiment.

(6-2-3) Summary

As described above, the signal processing device 10 of the second modification may determine the patient's hearing environment for the sound corresponding to the output acoustic signal, and generate target information further based on the determination result. This makes it possible to notify related parties of information regarding the patient's listening environment, and to notify them of a score calculated taking into account the effect that the patient's listening environment has on improving cognitive function.

(7) Other Modifications

The storage 11 may be connected to the signal processing device 10 via a network. The display 21 may be integrated with the signal processing device 10. The storage 71 may be connected to the related party terminal 70 via a network. The display 81 may be integrated with the related party terminal 70.

Each step of the above information processing can be executed by either the signal processing device 10 or the related party terminal 70. Some of the above information processing steps may be executed by the related party terminal 70 or an external device (e.g., a server) not shown. For example, the signal processing device 10 may store the listening history in an external device, the external device may generate target information based on the listening history, and the signal processing device 10 may acquire and notify the target information generated by the external device. In this case, the signal processing device 10 may omit the process of S120 in FIG. 7, and obtain the score from the external device in S121.

In the above description, an example in which the signal processing device 10 is connected to one audio output device 30 has been shown. The signal processing device 10 may be connected to a plurality of audio output devices 30, and in this case, the signal processing device 10 may be configured to be able to select to which audio output device 30 the output sound signal is to be sent.

In the above description, an example in which the signal processing device 10 is connected to one sound source device 50 has been shown. The signal processing device 10 may be connected to a plurality of sound source devices 50, and in this case, the signal processing device 10 may be configured to be able to select from which sound source device 50 the input sound signal is to be acquired.

The above description has focused on the case where one signal processing device 10 is associated with one patient. However, the present invention is not limited to this, and a plurality of patients may be associated with one signal processing device 10. For example, consider a case where a signal processing device 10 and a speaker serving as the audio output device 30 are installed in a room in which a plurality of patients (users who listen to the sound output from the audio output device 30) are present. In this case, the signal processing device 10 identifies the multiple patients in the room and updates the listening history database corresponding to each patient based on the acoustic signal output result by the signal processing device 10. As a result, even when a plurality of patients listen to a sound based on an acoustic signal output from one signal processing device 10, it is possible to easily grasp the listening status of each patient. One method for the signal processing device 10 to identify a patient who is listening to the sound emitted from the audio output device 30 (e.g., a patient who is in the same room as the audio output device 30) is to obtain ID information registered when the patient enters the room. In addition, for example, there is a method of identifying a patient who is in the vicinity of the audio output device 30 by performing wireless communication such as Bluetooth between a terminal device carried by the patient and the audio output device 30. However, the present invention is not limited to these, and the signal processing device 10 can use various existing identification methods.

Furthermore, a plurality of signal processing devices 10 may be associated with one patient. For example, consider a case in which the signal processing device 10 and a speaker serving as the audio output device 30 are installed in each of a number of rooms in a facility where patients live. In this case, multiple signal processing devices 10 within the facility access a common listening history database. Then, each signal processing device 10 identifies a patient who is in the same room as the audio output device 30 connected to that signal processing device 10, and updates the listening history database corresponding to that patient based on the output result of the audio signal by that signal processing device 10. As a result, even when one patient listens to sounds based on acoustic signals output from a plurality of signal processing devices 10, it is possible to easily grasp the listening condition of the patient. The signal processing device 10 can use the various methods described above to identify the patient.

Similarly, a plurality of signal processing devices 10 may be associated with a plurality of patients. That is, each of the multiple signal processing devices 10 may update a listening history database for each of the multiple patients who listen to sounds based on the acoustic signals output from that signal processing device 10. The signal processing device 10 may then transmit a notification based on the information obtained from the listening history database to the related party terminal 70 associated with each patient.

The signal processing device 10 may be equipped with a function for conducting cognitive function tests (for example, a function for measuring cognitive function through voice dialogue with a patient). In this case, the signal processing device 10 may notify the related party terminal 70 of target information including information indicating at least one of the patient's answers to the cognitive function test and the results of the patient's cognitive function test. This allows related parties to understand the patient's hearing status as well as the patient's cognitive function in a timely manner.

In the second modification, an example is shown in which the score is calculated taking into account the listening environment information. The signal processing device 10 may calculate the score taking other information into consideration.

As a first example, the signal processing device 10 may acquire information regarding the attributes of a patient who hears a sound corresponding to the output acoustic signal (hereinafter referred to as "attribute information"), and calculate a score further based on the attribute information. The attribute information may include, for example, at least one of the patient's age, sex, hearing ability (which may include hearing in different bands or the ability to hear speech), dementia risk, past listening history (which may include the results of an analysis of past listening history), or preferences or intentions (commitment) regarding listening to sound, the results of past electroencephalography or psychological experiments, or features based on those results. This makes it possible to obtain a score that takes into account the influence that patient attributes have on the improvement of cognitive function. The signal processing device 10 may use a sensor 90 (particularly, a device constituting a radio wave positioning system, or a camera) to identify the patient. In order to measure the hearing ability of a patient, a hearing test function may be implemented in the signal processing device 10 or the related party terminal 70.

As a second example, the signal processing device 10 may obtain the results of a cognitive function test of a patient who listens to a sound corresponding to the output acoustic signal (i.e., the patient's current cognitive function), and calculate a score further based on the results of the test. This makes it possible to obtain a score that takes into account the influence that the patient's current cognitive function has on the effect of improving cognitive function. The signal processing device 10 or the related party terminal 70 may be equipped with a function for conducting a cognitive function test (for example, a function for measuring cognitive function through voice dialogue with the patient).

The signal processing device 10 may change the method of generating the output acoustic signal (e.g., the modulation method, the synthesis ratio of two or more acoustic signals, the method of determining the acoustic signal to which amplitude modulation is applied, etc.) or the playback volume of the sound corresponding to the output acoustic signal depending on the patient's score. As a first example, the signal processing device 10 may generate an output audio signal corresponding to a sound for which a higher score can be obtained by listening, or may increase the playback volume, the shorter the time remaining until the expiration of the period for which the target score is set. As a second example, the signal processing device 10 may generate an output sound signal corresponding to a sound that can be listened to and that can achieve a higher score, or may increase the playback volume, as the patient's current achievement level decreases. In addition, if the score achievement does not meet a predetermined standard, the signal processing device 10 may automatically play an amplitude-modulated acoustic signal, or may play a predetermined acoustic signal that has a large brain wave eliciting effect (for example, a beep or chirp sound that is unrelated to the input acoustic signal and has a frequency corresponding to gamma waves). This makes it possible to assist the patient in achieving the target score without the patient having to pay attention to the score they are achieving.

In the above description, an example has been given in which the score is calculated based on the listening history stored by the signal processing device 10. However, in addition to or instead of such listening history, a score may be calculated based on the audio signal received by the microphone (e.g., the intensity, duration, waveform, or a combination thereof of the audio signal). Such a microphone may be installed closer to the patient's listening position than the audio output device 30, and may be, for example, a microphone provided on a terminal used by the patient, or a microphone that can be worn on the patient's body (for example, near the ear).

The algorithm for determining how to generate an output audio signal (e.g., the modulation method, the combining ratio of two or more audio signals, the method for determining the audio signal to which amplitude modulation is applied, etc.), or the default method for generating an output audio signal, may be changed by updating the firmware of the signal processing device 10. The firmware is updated, for example, by the signal processing device 10 communicating with an external device (for example, a server not shown). Similarly, the method of generating the target information (which may include the method of calculating the score), the method of notifying the target information, the method of presenting the target information, and the like may be changed by updating the firmware of the signal processing device 10. The contents of the update of the firmware of the signal processing device 10 may be notified to the related parties in advance, and the signal processing device 10 may provide an operation mode that enables the related parties to compare sounds before and after the update by listening to them.

Although an example has been given in which the input sound signal is acquired from the sound source device 50, the input sound signal may be generated by receiving environmental sound by a microphone. The input acoustic signal may be read from the storage 11 or a removable medium connected to the signal processing device 10. The sound source device 50 (including the sound source device 50 built into the signal processing device 10) may generate the input sound signal each time.

In the above description, an example has been given in which an audio signal derived from an input audio signal is modulated. However, the output audio signal may also be generated based on an input audio signal and a modulated audio signal that is not derived from the input audio signal.

The above description has focused on an example in which the modulation function has a frequency of 35 Hz or more and 45 Hz or less. However, the modulation function used by the signal processing device 10 is not limited to this, and any modulation function that has an effect on the induction of gamma waves in the listener's brain may be used. For example, the modulation function may have a frequency between 25 Hz and 140 Hz. Also for example, the frequency of the modulation function may vary over time, with portions of the modulation function having frequencies below 35 Hz or above 45 Hz.

In the above description, the output audio signal generated by the signal processing device 10 is output to the audio output device 30 that emits sound to be heard by the patient. However, the destination of the output sound signal from the signal processing device 10 is not limited to this. For example, the signal processing device 10 may output the output acoustic signal to an external storage device or information processing device via a communication network or by broadcasting. At this time, the signal processing device 10 may output, to an external device, an input acoustic signal that has not been subjected to modulation processing, together with an output acoustic signal generated by modulation processing. This allows the external device to arbitrarily select and play back either an unmodulated audio signal or a modulated audio signal. Furthermore, the signal processing device 10 may output information indicating the content of the modulation process to an external device together with the output acoustic signal. The information indicating the content of the modulation process includes, for example, any of the following:

- Information indicating the sound source corresponding to the modulated acoustic signal
- Information indicating the channel corresponding to the modulated audio signal
- Information indicating the characteristics of the modulated acoustic signal
- Information indicating the modulation function
- Information indicating modulation depth
- Information indicating volume

This allows the external device to change the method of reproducing the audio signal depending on the content of the modulation process.

In the above description, an example has been shown in which the signal processing device 10 performs modulation processing including amplitude modulation to generate a modulated acoustic signal. However, the signal processing device 10 may also obtain a modulated acoustic signal generated by an external device, and output an output acoustic signal based on the modulated acoustic signal. In this case, the signal processing device 10 may acquire information required for generating a listening history (for example, modulation method information or feature information) from an external device.

The related party terminal 70 may collect other information (e.g., TV viewing status (type of program, viewing time, etc.), patient vital data obtained from a wearable device) and present the collected information together with the target information.

Furthermore, when the signal processing device 10 acquires additional information (for example, an ID3 tag in an MP3 file) together with the input audio signal, the signal processing device 10 may modify the additional information and output it to an external device together with the output audio signal.

Although the embodiments of the present invention have been described in detail above, the scope of the present invention is not limited to the above-described embodiments. Furthermore, the above-described embodiment can be improved or modified in various ways without departing from the spirit and scope of the present invention. Furthermore, the above-described embodiments and modifications can be combined.

[Reference Signs List]

1: Sound system
2: Sound system
10: Signal processing device
11: Storage device
12: Processor
13: Input/Output Interface
14: Communication interface
21: Display
30: Audio output device
50: Sound source device
70: Related party terminal
71: Storage device
72: Processor
73: Input/Output Interface
74: Communication interface
81: Display
90: Sensor

**Claims**

1.  A signal processing apparatus, comprising:

    an output means for outputting an acoustic signal generated by amplitude modulation, the acoustic signal having an amplitude change corresponding to a frequency of gamma waves;
    a storage means for storing information on the acoustic signal output by the output means; and
    a transmitting means for transmitting a notification based on the information stored in the storage means to an apparatus external to the signal processing apparatus.

2.  The signal processing apparatus according to claim 1, wherein
    the transmitting means transmits the notification to at least one of an apparatus owned by a user who listens to the acoustic signal output by the output means, an apparatus owned by a family member of the user, an apparatus owned by a caregiver of the user, and an apparatus owned by a medical professional.

3.  The signal processing apparatus according to claim 1, wherein
    the information on the acoustic signal stored in the storage means includes information indicating at least one of a period during which the acoustic signal was output by the output means, a modulation method for the output acoustic signal, a magnitude of the output acoustic signal, and a feature of the output acoustic signal.

4.  The signal processing apparatus according to claim 1, wherein
    the notification transmitted by the transmitting means includes information indicating at least one of an output history of the acoustic signal by the output means and a score determined based on the output history of the acoustic signal.

5.  The signal processing apparatus according to claim 1, wherein
    the transmitting means transmits the notification in response to the fact that at least one of an output history of the acoustic signal by the output means and a score determined based on the output history of the acoustic signal satisfies a predetermined condition.

6.  The signal processing apparatus according to claim 1, further comprising:

    a means for acquiring the results of a cognitive function test,
    wherein the transmitting means transmits the notification in response to the fact that the result of the cognitive function test acquired by the acquisition means satisfies a predetermined condition.

7.  The signal processing apparatus according to claim 1, wherein
    the transmitting means transmits, together with the notification, information indicating at least one of answers to a cognitive function test and a result of the cognitive function test.

8.  The signal processing apparatus according to claim 1, wherein
    the acoustic signal output by said output means has an amplitude change corresponding to a frequency between 35 Hz and 45 Hz.

9.  A cognitive function improvement system comprising:

    the signal processing apparatus according to claim 1 to 8; and
    a means for making a user hear a sound corresponding to the acoustic signal output by the signal processing apparatus.

10. A signal processing method performed by a signal processing apparatus, comprising:

outputting an acoustic signal generated by amplitude modulation, the acoustic signal having an amplitude change corresponding to a frequency of gamma waves;
storing information about the output acoustic signal; and
transmitting a notification based on the stored information to an apparatus external to the signal processing apparatus.

11. A program for causing a computer to function as each of the means of the signal processing apparatus according to claim 1 to 8.

FIG. 1

1

```
        ┌─────────────────┐
        │   Sound source  │
        │      device     │
        │        50       │
        └────────┬────────┘
                 │
                 │
┌─────────────────┐         ┌─────────────────┐
│ Signal Processing│         │  Related party  │
│      Device     ├─────────┤    terminal     │
│        10       │         │        70       │
└────────┬────────┘         └─────────────────┘
         │
         │
┌─────────────────┐
│  Audio output   │
│      device     │
│        30       │
└─────────────────┘
```

FIG. 2

```
                                    ┌──────────────────┐
                                    │     display      │
                                    │        21        │
                                    └──────────────────┘
                                             │
  ┌──────────────────────────────────────────────────────────────┐
  │ 10                                        │                    │
  │      ┌──────────────────┐       ┌──────────────────┐          │
  │      │     Storage      │       │  Input/Output    │          │
  │      │       11         │       │   interface      │          │
  │      │                  │       │       13         │          │
  │      └──────────────────┘       └──────────────────┘          │
  │               │                          │                    │
  │      ─────────┼──────────────────────────┼──────────          │
  │               │                          │                    │
  │      ┌──────────────────┐       ┌──────────────────┐          │
  │      │    Processor     │       │  communication   │          │
  │      │       12         │       │   interface      │          │
  │      │                  │       │       14         │          │
  │      └──────────────────┘       └──────────────────┘          │
  └──────────────────────────────────────────────────────────────┘
```

FIG. 3

display
81

70

Storage
71

Input/Output
interface
73

Processor
72

communication
interface
74

FIG. 4

50

(1) Acquisition of acoustic signals

10

(2) Amplitude modulation

30

(3) Sound signal output

(4) Storage of listening history
(5) Creation of target information

PA1

70

PC2

(5) Notification of target information

FIG. 5

| Listening history database | | | | | |
|---|---|---|---|---|---|
| ID | Start date and time | End date and time | Modulation Method | Features | Playback volume |
| H...1 | Apr 12, 2022 9:17 | Apr 12, 2022 10:01 | A1 | B2 | C3 |
| H...2 | Apr 12, 2022 11:31 | Apr 12, 2022 11:47 | A2 | B3 | C1 |
| H...3 | . | . | . | . | . |

FIG. 6

```
        ┌─────────────────────┐
        │        START        │
        └─────────────────────┘
                   │
                   │                    S110
        ┌─────────────────────┐
        │ Acquiring the input │
        │  acoustic signal    │
        └─────────────────────┘
                   │
                   │                    S111
        ┌─────────────────────┐
        │ Generation of output│
        │   acoustic signal   │
        └─────────────────────┘
                   │
                   │                    S112
        ┌─────────────────────┐
        │ Sending the output  │
        │   audio signal      │
        └─────────────────────┘
                   │
                   │                    S113
        ┌─────────────────────┐
        │ Saving listening    │
        │      history        │
        └─────────────────────┘
                   │
                   ▼
        ┌─────────────────────┐
        │         END         │
        └─────────────────────┘
```

FIG. 7

START

Acquiring listening history — S120

Generation of target
information — S121

Notification of target
information — S122

END

FIG. 8

Modulation method A2, feature quantity B1

Modulation method A1, feature quantity B2

Modulation method A1, feature quantity B1

Score

Playback volume C1

Playback volume C2

Listening period

FIG. 9

P80

[List of patients in charge]

・ Yamada Taro ▷
・ Jiro Suzuki ▷
・ Hanako Kato ▷
・
・
・

A801

click

P81

[Personal screen] Yamada Taro

・ Patient information ▷ ——A811

・ score display ▷ ——A812

・ Cognitive function
  test results ▷ ——A813

・ Display medical records ▷ ——A814

FIG. 10

P82

## Taro Yamada

### 2022-X-18

A821

85/100

Today's achievement

A822

Detailed History

A823

127
95  108  86        63 85
77        25          100

7 days ago          today

Achievement History

year    month    week

A824

Doctor Comment History

A825

Cognitive function test results

A826

Statistics

A827

Send us a message

FIG. 11

P83

**Taro Yamada**

**2022-X-17**

A831

63/100

Today's achievements

A833

| ←Previous day |

A834

A832

| Next day → |

High efficiency | Low efficiency | Processing OFF | ☐ Sound Off

100
75
50
25

0h          6h          12h          18h          24h

Cumulative achievement

FIG. 12

```
                    ┌─────────────────────┐
                    │        START        │
                    └─────────────────────┘
                               │
                               ▼                        S120
          ┌─────────────────────────────────┐
          │      Acquiring listening        │
          │            history              │
          └─────────────────────────────────┘
                               │
                               ▼                        S121
          ┌─────────────────────────────────┐
          │      Generation of target       │
          │          information            │
          └─────────────────────────────────┘
                               │
                               ▼                        S223
          ┌─────────────────────────────────┐
          │    Determining notification     │
          │          conditions             │
          └─────────────────────────────────┘
                               │
                               ▼
                          ╱         ╲
          NO           ╱   Notification  ╲
    ◄───────────────  ╱    conditions     ╲
                      ╲  Establishment of? ╱
                       ╲                  ╱
                          ╲           ╱
                               │ YES
                               ▼                        S122
          ┌─────────────────────────────────┐
          │     Notification of target      │
          │          information            │
          └─────────────────────────────────┘
                               │
                               ▼
                    ┌─────────────────────┐
                    │         END         │
                    └─────────────────────┘
```

FIG. 13

2

```
                    ┌──────────────┐
                    │ Sound source │
                    │   device     │
                    │     50       │
                    └──────┬───────┘
                           │
┌──────────────┐   ┌───────┴──────────┐   ┌──────────────┐
│   Sensors    │   │ Signal Processing│   │ Related party│
│      90      ├───┤     Device       ├───┤   terminal   │
│              │   │       10         │   │     70       │
└──────────────┘   └───────┬──────────┘   └──────────────┘
                           │
                    ┌──────┴───────┐
                    │ Audio output │
                    │   device     │
                    │     30       │
                    └──────────────┘
```

FIG. 14

```
         ┌─────────────────┐
         │      START      │
         └────────┬────────┘
                  │                      S110
         ┌────────┴────────┐
         │ Acquiring the input acoustic │
         │      signal     │
         └────────┬────────┘
                  │                      S111
         ┌────────┴────────┐
         │  Generation of output │
         │  acoustic signal │
         └────────┬────────┘
                  │                      S112
         ┌────────┴────────┐
         │  Sending the output audio │
         │      signal     │
         └────────┬────────┘
                  │                      S314
         ┌────────┴────────┐
         │  Judging the listening │
         │   environment   │
         └────────┬────────┘
                  │                      S113
         ┌────────┴────────┐
         │ Saving listening history │
         └────────┬────────┘
                  │
         ┌────────┴────────┐
         │       END       │
         └─────────────────┘
```

## INTERNATIONAL SEARCH REPORT

| International application No. |
|---|
| **PCT/JP2023/023525** |

### A. CLASSIFICATION OF SUBJECT MATTER

***G16H 10/00***(2018.01)i
FI:   G16H10/00

According to International Patent Classification (IPC) or to both national classification and IPC

### B. FIELDS SEARCHED

Minimum documentation searched (classification system followed by classification symbols)

G16H10/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2018-156485 A (RICOH CO., LTD.) 04 October 2018 (2018-10-04)<br>claim 1, paragraphs [0013], [0029], [0033], [0037], [0039]-[0040], [0050], [0070]-[0072], [0078], [0080] | 1-5, 7-11 |
| A | JP 2008-48757 A (SONY CORP.) 06 March 2008 (2008-03-06)<br>entire text | 1-11 |
| A | JP 2020-501853 A (COGNITO THERAPEUTICS, INC.) 23 January 2020 (2020-01-23)<br>entire text | 1-11 |

☐ Further documents are listed in the continuation of Box C.       ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **30 August 2023** | **12 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2023/023525**

| Patent document cited in search report | | | Publication date (day/month/year) | Patent family member(s) | | | Publication date (day/month/year) |
|---|---|---|---|---|---|---|---|
| JP | 2018-156485 | A | 04 October 2018 | (Family: none) | | | |
| JP | 2008-48757 | A | 06 March 2008 | US<br>entire text | 2008/0051919 | A1 | |
| JP | 2020-501853 | A | 23 January 2020 | US<br>entire text | 2018/0133431 | A1 | |
| | | | | WO | 2018/094226 | A1 | |
| | | | | EP | 3541467 | A1 | |
| | | | | AU | 2017363200 | A1 | |
| | | | | CA | 3044440 | A1 | |
| | | | | KR | 10-2019-0097032 | A | |

Form PCT/ISA/210 (patent family annex) (January 2015)

36

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020501853 A

**Non-patent literature cited in the description**

- *Multi-sensory Gamma Stimulation Ameliorates Alzheimer's-Associated Pathology and Improves Cognition Cell*, 04 April 2019, vol. 177 (2), 256-271